# FASCICULE DE BREVET EUROPEEN

(11) **EP 0 843 738 B1**
(45) Date de publication et mention de la délivrance du brevet: **12.01.2000**
(21) Numéro de dépôt: 96927108.9
(22) Date de dépôt: 30.07.1996
(51) Int. Cl.: C12Q 1/68

(54) **PROCEDE DE DETECTION D'ACIDES NUCLEIQUES UTILISANT DES SONDES NUCLEOTIDIQUES PERMETTANT A LA FOIS UNE CAPTURE SPECIFIQUE ET UNE DETECTION**
VERFAHREN ZUR DETEKTION EINER NUKLEINSAEURE MITTELS EINER NUKLEINSAEURESONDE, DAS EIN SPEZIFISCHES EINFANGEN UND EINE DETEKTION AUF EINMAL ERLAUBT
NUCLEIC ACID DETECTION METHOD USING NUCLEOTIDE PROBES ENABLING BOTH SPECIFIC CAPTURE AND DETECTION

(30) Priorité: 31.07.1995 FR 9509290
(43) Date de publication de la demande: 27.05.1998
(73) Titulaire: GENSET SA, 75008 Paris (FR)
(72) Inventeur: BLUMENFELD, Marta, F-75013 Paris (FR); BOUILLOT, Michel, F-91860 Epinay-sous-Senart (FR)
(74) Mandataire: Warcoin, Jacques
(86) Numéro de dépôt international: FR9601201
(87) Numéro de publication internationale: WO9705277

(56) Documents cités:
- EP-A- 0 622 464
- WO-A-94/26934
- WO-A-95/11316
- MOL. AND CELL. PROBES, vol. 5, no. 3, Juin 1991, ACADEMIC PRESS, NEW YORK, US, pages 161-205, XP002002944 C. KESSLER : "The digoxigenin: anti-digoxigenin (DIG) technology a survey on the concep and realization of a novel bioanylytical indicator system"
- BIOCHEMICA, NEWSLETTER FROM BOEHRINGER MANNHEIM, vol. 1, 1994, MANNHEIM, BRD, pages 13-15, XP002002945 A. VIAENE AND J. BAERT: "In situ hybridization of DIG-labeled RNA probes in a study of cytokeratin expression in human esophageal epithelium"
- BIOCHEMICA, NEWSLETTER FROM BOEHRINGER MANNHEIM, vol. 1, 1994, MANNHEIM, BRD, pages 4-5, XP002002946 "PCR ELISA"

## Description

La présente invention concerne un procédé de détection d'acides nucléiques utilisant des sondes nucléotidiques permettant à la fois une capture spécifique et une détection. La présente invention permet l'automatisation de la détection spécifique à grande échelle des acides nucléiques en utilisant une sonde ribonucléotidique simple brin complémentaire à une séquence nucléotidique cible, ladite sonde permettant à la fois la capture sur une surface appropriée et une détection rapide et sensible.

Par le biais des grands programmes de séquençage et d'analyse des génomes, la biologie est rentrée depuis quelques années dans une ère nouvelle, celle de l'acquisition massive de données, basée sur l'utilisation intensive de techniques existantes améliorées. D'ici cinq à dix ans, le génome de plusieurs organismes modèles ainsi, que le génome humain seront totalement séquences. Le nombre de gènes codés par le génome humain étant calculés entre 50.000 et 100.000, plusieurs milliers de séquences de régions codantes (cDNA) ainsi que de régions régulatrices (promoteurs) devront être répertoriées, analysées et intégrées dans des bases de données, et leurs profils d'expression dans une centaine de tissus ou cellules pourront être établis.

Le profil d'expression d'un gène consiste en l'étude de l'abondance relative de l'ARN messager (ARNm) correspondant exprimé dans des cellules particulières (par example, appartenant à des tissus différents, ou répresentant differents stades du developement, des pathologies particulières, des inductions par des drogues, etc). L'établissement des profils d'expression des gènes requiert donc la détection spécifique et sensible des ARNm.

La détection des ARNm spécifiques peut être entreprise par deux types d'approches différentes: celles basées sur des techniques d'amplification et celles basées sur des techniques d'hybridation.

L'utilisation de techniques d'amplification, tel que la RT-PCR (Reverse transcriptase-Polymerase Chain Reaction), apparait peu utilisable dans un projet d'étude de profils d'expression d'ARNm à grande échelle du fait du coût que demanderait la synthèse du grand nombre d'oligonucléotides nécessaires à sa réalisation (pour 50.000 gènes différents, le nombre minimum serait de 100.000 oligonucléotides).

La technique d'hybridation "sandwich" décrite pour la première fois par Dunn A.R. et Hassell J.A. (1977, cell, 12, 23-36) a été développée pour éviter la purification et l'immobilisation d'acides nucléiques cibles qui nécessitaient auparavant une détection après une hybridation en phase solide. Cette technique est basée sur l'existence de deux sondes, non chevauchantes, dirigées contre le même acide nucléique cible. La première sonde est immobilisée sur une surface solide, et permet la capture de la cible. La deuxième sonde possède un traceur dans sa séquence, et permet la détection de la cible. Cette technique est essentiellement utilisée pour la détection de produits d'amplification. Effectivement les sondes utilisées sont de petites tailles (de 20 à 25 nucléotides) et à la différence de sondes longues, elles ne permettent pas l'obtention de forts signaux d'hybridation. Cette technique est peu adaptée à la détection simultanée d'un nombre important de cibles, car chaque sonde nécessite des températures d'hybridation qui peuvent être différentes.

La deuxième approche est basée sur des techniques d'hybridation ne faisant pas appel à une amplification, et qui requièrent donc une grande sensibilité. Il existe plusieurs formats qui peuvent être utilisés pour détecter une hybridation spécifique: l'hybridation en phase liquide; l'hybridation en phase solide; l'hybridation in situ sur des tissus ou corps cellulaires. Dans tous les cas, la sonde est un acide nucléique (ADN ou ARN) capable de s'hybrider à une séquence d'acide nucléique complémentaire: la cible (ADN ou ARN). Les cinétiques des différentes réactions d'hybridation des acides nucléiques sont bien connues (Britten et al., 1974, Methods Enzymol, 29, p.363; Kohme et al., 1977 Biochemistry, 16, p.5329-5341), et la connaissance des paramètres impliqués dans le taux d'hybridation ainsi que dans la stabilité des hybrides cible-sonde permet de déterminer les conditions optimales pour obtenir le meilleur rapport signal sur bruit.

L'hybridation en phase liquide est très efficace et a l'avantage d'offrir le taux d'hybridation le plus rapide. Cependant, il est difficile de séparer la sonde libre de la sonde hybridée. Les méthodes d'hybridation en phase solide qui impliquent l'immobilisation de la cible (ou de la sonde) sur une surface solide (ex : nitrocellulose, nylon) sont plus faciles à mettre en oeuvre du point de vue de la séparation de la sonde non hybridée. Cependant, par rapport à l'hybridation en solution, le taux d'hybridation est de 7 à 10 fois plus lent. Enfin, l'hybridation in situ permet l'examen microscopique de séquences ADN ou ARN présent dans des cellules ou des tissus tout en préservant leurs localisations. C'est une méthode appropriée aux laboratoires de cytologie ou d'histologie.

Dans le cas précis de la détection des ARN, la technique en phase solide dit du "Northern blot" constitue la méthode d'hybridation la plus répandue, utilisée aussi bien en analyse médicale qu'en recherche fondamentale. Le "Northern blot", bien qu'étant la seule méthode capable de fournir des informations sur la taille des messagers, et donc, sur l'identité de la cible, ne peut pas être considerée pour des analyses à grande échelle à cause de sa faible sensibilité et des difficultés d'automatisation. Pour des raisons similaires, la technique d'hybridation in situ ne parait pas non plus appropriée à des études de profil d'expression d'ARN à grande échelle.

Parmi les méthodes de détection d'ARN basées sur l'hybridation en solution, la technique de protection aux nucléases présente l'intêret d'être une méthode très sensible et spécifique. Dans cette technique, des sondes radioactives ARN ou ADN simple brin sont hybridés en solution avec des préparations d'ARN contenant l'ARN cible. Après hybridation, les acides nucléiques non hybridés (aussi bien la sonde non hybridé que les ARNs de la préparation) sont dégradés par action des nucléases spécifiques des acides nucléiques simple brin (en général, RNases A et T1 pour les sondes ARN, et nucléase S1 pour les sondes ARN ou ADN). L'hybridation de la sonde avec sa cible ARN complémentaire "protège" la sonde de la dégradation par la nucléase, et donne comme résultat des molécules double brin dont la longueur est définie par la complémentarité sonde-cible.

D'autre part, l'hybridation non spécifique de la sonde avec des ARN autres que la cible, donne comme résultat des molécules double brin plus courtes que celles provenant de l'hybridation spécifique. Les deux types de produits, spécifique et non-spécifique, peuvent donc être distingués selon la taille, l'analyse la plus habituelle étant l'electrophorèse en gel de polyacrylamide. Une technique alternative d'analyse par séparation chromatographique a été proposé dans WO 95/11316.

Si la technique de protection aux nucléases présente l'avantage d'être sensible et spécifique, la méthode analytique consistant à une séparation électrophorétique ou chromatographique est incompatible avec une automatisation de la méthode et l'utilisation d'un grand nombre d'échantillons.

La présente invention est destinée à permettre une détection spécifique d'acides nucléiques, notamment d'ARN dans un format qui rende possible la manipulation simultanée d'un grand nombre d'échantillons par une procédure entièrement automatisable, tout en étant sensible, spécifique et reproductible.

Un autre but de la présente invention est en particulier de fournir une méthode qui permette la détection spécifique des ARNm à partir des ARN totaux.

L'invention repose sur l'utilisation d'une méthode d'hybridation en solution des acides nucléiques avec une sonde nucléotidique qui, en plus d'une spécificité de reconnaissance apportée par sa séquence, possède des modifications lui permettant d'accomplir deux fonctions différentes. La première fonction permet la fixation à un support solide de l'hybride sonde-cible ou de la sonde provenant d'un hybride sonde-cible préalablement dénaturé. La deuxième fonction de la sonde permet une détection spécifique par des méthodes de détection radioactive ou froide.

De façon générale l'invention implique une méthodologie comprenant:
(1) une hybridation en phase liquide utilisant une sonde d'acide nucléique doublement marquée s'hybridant spécifiquement avec un ARN ou un ADN donné.
(2) une dégradation par des nucléases des séquences nucléotidiques non hybridées.
(3) la séparation des hybrides par une capture spécifique sur un support solide des hybrides formés, par l'intermédiaire d'une modification de la sonde.
(4) une détection des hybrides grâce à la modification précité, ou par l'intermédiaire d'une autre modification présent sur la sonde.

Plus précisément la présente invention a pour objet un procédé de détection d'acides nucléiques caractérisé en ce qu'il comporte les étapes suivantes :
a) on réalise l'hybridation en solution d'une sonde d'acide nucléique de grande taille, notamment de plus de 100 nucléotides, capable de s'hybrider avec un ARN ou un ADN donné dans un échantillon contenant des acides nucléiques, ladite sonde comprenant des éléments de détection et des premiers éléments de capture sur support solide;
b) on réalise la dégradation des séquences nucléotidiques non hybridées dans l'échantillon par un enzyme dégradant les acides nucléiques non hybridées,
c) on effectue la capture des hybrides obtenus à l'étape b) en les mettant en présence d'un support solide revêtu des deuxièmes éléments de capture interagissant avec lesdits premiers éléments de capture de ladite sonde, et
d) on effectue la détection des hybrides ou des sondes provenant des hybrides après dénaturation, par l'intermédiaire desdits éléments de détection.

L'utilisation de sondes de taille importante, notamment de plus de 100 nucléotides, confère une plus grande spécificité d'hybridation et la possibilité d'introduire des modifications suffisantes pour la détection et la capture de la sonde tout en conservant la stabilité de l'hybridation.

En particulier la sonde comporte de 100 à 1000 nucléotides, de préférence de 250 à 500 nucléotides.

Un aspect de l'invention est lié à l'utilisation, après incubation avec des nucléases, d'une ou plusieurs techniques d'élimination d'oligonucléotides (moins de 100 bases), tels que la precipitation différentielle, la filtration à travers des membranes, la filtration sur gel (chromatographie d'exclusion), ou toute autre technique permettant la séparation des hybrides cible-sonde des produits de dégradation des acides nucléiques notamment de la sonde non hybridée.

En général, les deux fonctions de la sonde sont accomplis par deux modifications nucléotidiques différentes. Cependant, il est à noter que la même modification peut être utilisée pour la capture et pour la détection.

Selon la présente invention, la sonde comprend une quantité suffisante d'éléments de détections et d'éléments de capture pour assurer une capture puis une détection suffisante. Toutefois, la quantité desdits éléments de détection et desdits premiers éléments de capture fixés sur la sonde est optimisée pour être compatible avec l'hybridation de l'étape a) et la non dégradation des hybrides à l'étape b).

Il faut considérer que l'introduction de nucléotides modifiés dans les sondes bifonctionnelles peut provoquer une déstabilisation des hybrides spécifiques au niveau des appariements proches des modifications. Il ressort en effet des exemples illustrant l'invention décrits ci-après, que si la quantité des modifications de la sonde est trop importante, l'hybridation n'est pas suffisamment stable de sorte que même les hybrides peuvent se trouver dégradés par les enzymes utilisés à l'étape b).

De préférence, lorsque les éléments de détection et premiers éléments de capture sont des molécules biologiques substituées sur des nucléotides constituant la sonde, celle-ci ne doit pas comporter plus de 15 %, de préférence pas plus de 10 % de nucléotides modifiés par lesdits éléments de détection et lesdits premiers éléments de capture. On comprendra également que ces quantités sont dépendantes de la taille des molécules constituant les éléments de détection et éléments de capture. Si l'encombrement stérique est trop grand, ils fragiliseront la stabilité de l'hybridation.

Les sondes nucléotidiques selon la présente invention peuvent être des sondes ADN ou, de préférence, des sondes ARN. Les sondes ARN peuvent être obtenues par transcription in vitro et possèdent quelques avantages par rapport aux sondes ADN. Les sondes ARN se trouvent directement sous la forme d'un simple brin, ainsi elles ne nécessitent pas de dénaturation. D'autre part il n'existe pas d'interférence avec un brin complémentaire comme pour les sondes ADN. Aussi, les sondes ARN forment des hybrides plus stables avec leurs cibles que ceux obtenus avec les sondes ADN.

De façon avantageuse, pour détecter un ARNm exprimé à partir d'un gène donné, on utilise une sonde d'ARN obtenue par transcription in vitro de fragments d'ADNc clonés dudit gène.

De façon avantageuse, on utilise une sonde d'ARN obtenue par transcription in vitro à l'aide de nucléotides dont certains sont modifiés par couplage à undit élément de détection et/ou undit élément de capture.

De nombreuses modifications peuvent être incorporées dans les ARN transcrits par les ARN polymérases SP6, T3, et T7. Par exemple, des ribonucléotides comportant des groupements biotine (tels que biotine-UTP et DIG-UPT), ou fluorescéine (tels que F1-11-UTP, F1-12-UTP) existent qui peuvent remplacer l'UTP dans les réactions de transcription in vitro catalisées par les ARN polymérases SP6, T3, ou T7. Aussi, d'autres types de modifications peuvent être incorporées aux ribonucléotides selon les techniques connues de l'homme de l'art.

Après hybridation de la sonde bifonctionnelle avec la préparation contenant l'ARN cible, la sonde non hybridée doit être dégradée dans des conditions qui n'entrainent pas une dégradation des hybrides spécifiques sonde-cible. La méthode choisi pour éliminer la sonde non hybridée doit aussi tenir compte de la possible existence de régions simple brin dans l'hybride sonde-cible, afin de ne pas perdre une partie du signal spécifique. Il existe plusieurs enzymes ayant la capacité d'hydrolyser spécifiquement des acides nucléiques simple brin. Entre autres, on peut citer la nucléase S1, la nucléase de "mung bean" ou l'exonucléase VII, qui n'ont pas de spécificité de séquence, et la RNasc A, la RNase CL3, la RNase Phy M, la RNase T1 ou la RNase U2, qui coupent seulement en amont et/ou en aval de certains nucléotides. Fait partie de la présente invention l'utilisation d'une ou plusieurs enzymes nucléolytiques spécifiques des régions simple brin et n'étant pas spécifiques des nucléotides modifiés incorporés dans la sonde. Par exemple, si les ribosondes comportent des modifications liées à l'UTP, il faut de préférence utiliser la RNase T1 (qui coupe en 3' de G) et/ou la RNase CL3 (qui coupe en 3' de C) et/ou la RNase U2 (qui coupe en 3' de A), de façon à diminuer la probabilité de coupure autour des UTP modifiés dans l'hybride sonde-cible.

De façon avantageuse lorsque l'on détecte des ARNm à l'étape a) on utilise une sonde d'ARN et à l'étape b) on réalise la dégradation enzymatique avec une RNase.

Lorsque les éléments de détection et lesdits premiers éléments de capture sont substitués sur des nucléotides donnés, on utilise de préférence à l'étape b) un enzyme qui couple les acides nucléiques simple brin au niveau d'un nucléotide autre que lesdits nucléotides substitués.

En particulier les éléments de détection et lesdits premiers éléments de capture sont substitués sur des nucléotides d'Uridine et l'enzyme de l'étape b) est une RNase T₁.

Le groupement de capture permet une fixation spécifique de la sonde sur la phase solide.

Dans un mode de réalisation, lesdits premiers éléments de capture de ladite sonde sont constitués par une première molécule biologique fixée de façon covalente à ladite sonde et lesdits deuxièmes éléments de capture dudit support solide sont constitués par une deuxième molécule biologique fixée sur le support solide, deuxième molécule biologique qui interagit et se lie de façon non covalente avec ladite première molécule.

On cite en particulier le mode de réalisation dans lequel lesdites première et deuxième molécules constituent le couple biotine/streptavidine ou un couple antigène / anticorps ou encore plus particulièrement les premiers et deuxièmes éléments de capture peuvent être le couple digoxigénine (DIG) et un anticorps anti-DIG ou la fluorescéine et un anticorps anti-fluorescéine, plus généralement, n'importe quel couple ligand- récepteur ou haptène-anticorps.

Selon l'invention, la capture des acides nucléiques hybrides sonde-cible ou de la sonde provenant d'un hybride sonde-cible préalablement dénaturée est faite sur une surface solide. Cette surface est de préférence, mais ne se limite pas, à une microplaque. La capture peut être faite sur la surface d'un détecteur de résonance plasma (Surface plasmon résonance Fisher et al. 1994, Current Opinion in Biotechnology 5, 389-395). La surface choisie permet une fixation spécifique de la sonde grâce à l'immobilisation des molécules ayant une haute affinité pour les groupements de capture présents dans la sonde.

Le groupement de détection permet une mise en évidence spécifique de la sonde. Par exemple, la biotine peut être reconnue par la streptavidine ou par un anticorps anti-biotine couplé à un enzyme; la DIG par des anticorps anti-DIG couplés à un enzyme, ou la fluoresceine par des anticorps anti-fluorescéine couplés à un enzyme. La détection peut être également médiée par la présence d'un nucléotide radioactif dans la sonde.

L'élément de détection de la sonde peut être un élément directement ou indirectement détectable. En effet, on entend par "élément de détection" une molécule pouvant être détectée, directement ou indirectement c'est-à-dire dans le dernier cas après liaison par interaction ou couplage covalent avec une autre molécule et/ou une particule solide. Par "détection directe" on entend notamment les cas où ladite molécule comporte elle-même un élément détectable tel qu'un élément radioactif ou fluorescent, où ladite molécule est couplée à un enzyme que l'on peut détecter à l'aide d'un substrat ou encore ladite molécule est couplée à une molécule fluorescente. Par "détection indirecte", on entend notamment les cas où ladite molécule est une molécule biologique susceptible de réagir de manière physico-chimique par une interaction non covalente ou par couplage covalent avec une autre molécule biologique elle-même comportant un élément détectable directement tel qu'un atome radioactif ou fluorescent, un enzyme ou une molécule fluorescente.

L'élément de détection indirectement détectable peut être constitué par une molécule biologique susceptible de réagir de manière non covalente avec une autre molécule biologique comportant un élément directement détectable tel qu'un enzyme. On cite en particulier les couples streptavidine/biotine ou antigène/anticorps. En particulier, l'élément de détection indirectement détectable est choisi parmi la biotine, la fluoresceine ou la DIG.

Dans le cas de la détection radioactive, la présence d'un nucléotide radioactif dans la sonde peut être par exemple décelée par comptage dans un détecteur de radioactivité adaptée à des microplaques. Dans le cas de la détection froide, le groupement de détection incorporé dans la sonde à cet effet peut être reconnu à l'aide d'un ligand/anticorps spécifique conjugué à une enzyme, suivi d'une incubation avec un substrat. Ainsi, la biotine peut être reconnue par la streptavidine ou par un anticorps anti-biotine couplé à un enzyme; la DIG par des anticorps anti-DIG couplés à un enzyme, ou la fluoresceine par des anticorps anti-fluorescéine couplés à un enzyme. Les enzymes utilisées pour les détections froides peuvent être, entre autres, la phosphatase alcaline, la peroxidase ou la b galactosidase. Il existe pour ces enzymes de nombreux substrats colorimétriques, fluorescents ou chémoluminescents. La mesure d'activité enzymatique peut se faire par lecture automatique dans un colorimètre, fluorimètre ou luminomètre adapté aux microplaques.

Alternativement, si la surface de capture choisie est celle d'un biosensor de résonance de plasma, la présence de groupements de détection dans la sonde peut être déterminée par mesure directe de l'interaction avec des ligands/anticorps spécifiques.

Lorsque les éléments de capture et de détection sont des molécules biologiques substituées sur un nucléotide notamment de l'Uridine, on cite comme élément de détection ou de capture la biotine, la fluoresceine ou la digoxigénine. Plus particulièrement l'élément de détection est la digoxigénine et/ou la biotine et ledit premier élément de capture est choisi respectivement parmi la biotine ou la digoxigénine. Dans un mode de réalisation, ledit premier élément de capture est la biotine ou la digoxigénine, et les nucléotides portant ces modifications sont des Uridine et ledit élément de détection est la DIG ou la biotine respectivement.

De préférence, le groupement de détection est la DIG, le conjugué est un anticorps anti-DIG couplé à la phosphatase alcaline, et le substrat permet une détection chémoluminescente.

Dans un mode de réalisation illustré par les exemples de la description détaillée qui va suivre, la sonde comporte de 2 à 5% des nucléotides modifiés par couplage à undit élément de détection consistant en une molécule de biotine, notamment 3 à 4%, et de 2 à 5% des nucléotides modifiés par couplage à undit premier élément de capture consistant en une molécule de digoxigénine, notamment 2 à 3%.

La présente invention a également pour objet une méthode de diagnostic dans laquelle on utilise une méthode de détection selon la présente invention comprenant la détection d'acides nucléiques impliqués dans une pathologie.

La présente invention a également pour objet un kit utile pour la mise en oeuvre d'un procédé de détection ou de diagnostic selon la présente invention, caractérisé en ce qu'il comporte des sondes d'ARN de taille importante, notamment de plus de 100 nucléotides, portant des éléments de détection et desdits premiers éléments de capture sur support solide et un support solide revêtu desdits deuxièmes éléments de capture.

D'autres caractéristiques et avantages de la présente invention apparaîtront à la lumière de la description et des exemples qui vont suivre faisant référence aux figures 1 à 10.

La figure 1A représente les résultats de la fixation de 1 fmole de ribosonde biotine-DIG marquée au (32aP)CTP et permet de connaître le pourcentage de biotine mis à incorporer dans la ribosonde nécessaire à une bonne fixation sur plaque. Les ribosondes étudiées sont obtenues par transcription inverse à l'aide d'une proportion variable de biotine-UTP (5,10,15,25,100%) et d'une quantité fixe de DIG-UTP (10%). La détection des ribosondes est effectuée par mesure de la radioactivité.

La figure 1B représente la détection de 300 amoles de ribosondes à l'aide d'anticorps anti-DIG (sytème de détection froid). Les ribosondes étudiées sont constituées à partir d'un pourcentage variable de biotinc-UTP (5,10,15,25%), et de 10% de DIG-UTP.

La figure 2A montre la fixation de ribosondes biotine-UTP (25 et 100%) marquées au (α³²P)CTP avec une forte activité spécifique(2 10⁵ cpm/ fmole) . Ce qui permet de déterminer de façon plus précise la sensibilité de la fixation. Le ribosondes sont diluées de 2 en 2 à partir de 2,5 jusqu'à 0,01 fmoles.

La figure 2B montre les résultats de la détection de ribosondes biotine-DIG en fonction du pourcentage de DIG dans la ribosonde, en utilisant un anticorps anti-DIG couplé à la phosphatase alcaline suivi d'une mesure de fluorescence.

La figure 3A représente la capture de ribosondes (biotine-UTP) hybridées avec une séquence nucléotidiquc complémentaire marquée au (α³²P)CTP. Ce qui permet l'étude de la fixation des ribosondes sous forme double brin. Les ribosondes étudiées sont préparées à partir d'un pourcentage variable de biotine-UTP (5, 15, 25, 35%). Les hybrides sont dilués de 2 en 2 de 2 à 0,015 fmoles. La détection des hybrides est effectuée par mesure de la radioactivité. Ces courbes montrent l'optimum de biotine-UTP dans la ribosonde (sous forme double brin) pour obtenir une bonne fixation.

La figure 3B représente la capture de ribosondes (5,15,25,35% biotine-UTP) hybridées avec une séquence nucléotidique (35% DIG-UTP) marquée au (α³²P)CTP. Ce qui permet l'étude de la fixation des ribosondes sous forme double brin. La détection des hybrides est effectuée par mesure de la radioactivité.

La figure 4 montre l'influence du pourcentage de DIG-UTP sur l'importance du signal de détection à l'aide d'anticorps anti-DIG. Les sondes étudiées sont constituées de 15% biotine-UTP et de (5,15,20,25% de DIG-UTP).

La figure 5 montre l'utilisation de ribosondes DIG-UTP dans un test fonctionel de protection aux nucléases.

Les ribosondes étudiées sont constituées de DIG-UTP (0, 5,15, 25 et 35 %). Elles sont radiomarquées au (α³²P)CTP.

Chaque ribosonde est hybridée avec une séquence ribonucléotidique complémentaire (10 ou 1ng) en présence de 10 µg de ARN de levure (+). Il a été effectué pour chaque sonde un contrôle sans digestion par la Rnase T1 (-), et un contrôle RNase sans sonde complémentaire (0).

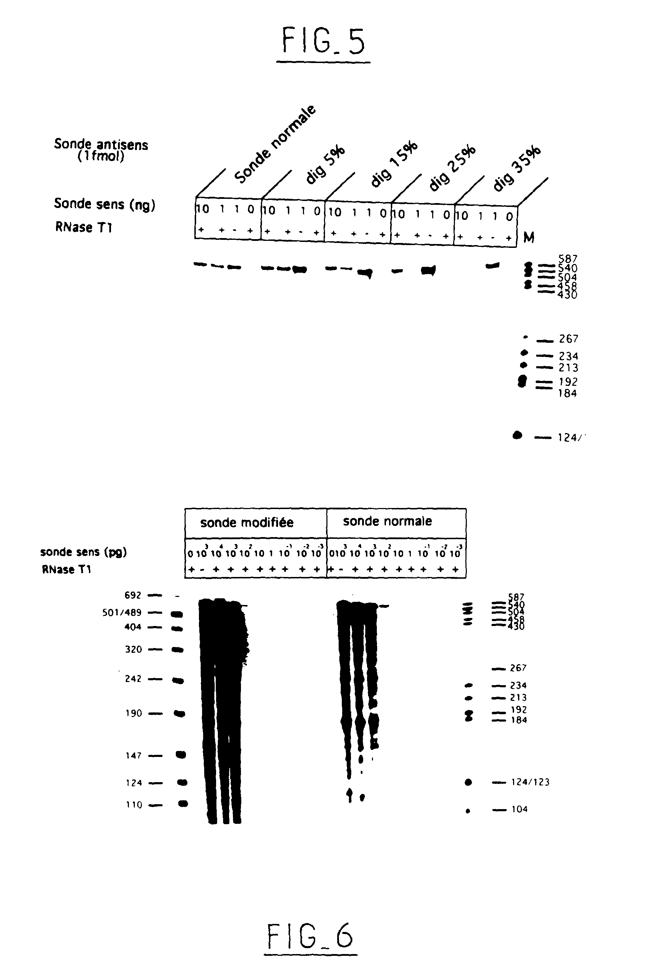

La figure 6 représente l'autoradiographie d'un gel de polyacrylamide et montre la sensibilité de détection à l'aide d'une ribosonde radiomarquée constituée de 15% de biotine-UTP et de 10% de DIG-UTP par rapport à la même ribosonde radiomarquée non modifiée.

Les deux ribosondes radiomarquées utilisées à 1 fmole (45000 cpm) ont été mises avec une quantité décroissante d'une séquence ribonucléotidique complémentaire froide (diluée de 10 en 10 à partir de 10⁴ à 10³ pg) en présence de 10 µg de ARN de levure (+). Il a été effectué pour chaque sonde un contrôle sonde sans digestion par la Rnase T1 (-), et un contrôle RNase sans sonde complémentaire (0). La bande visible sur le gel correspond à la ribosonde qui n'a pas été dégradée (car sous forme d'hybride) lors de la digestion par la Rnase T1.

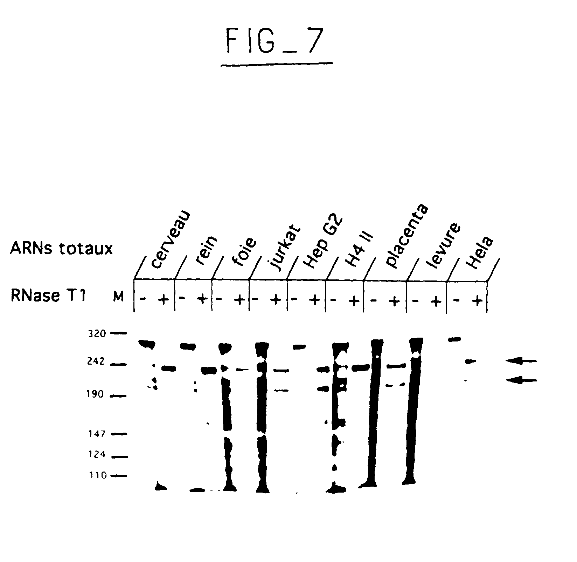

La figure 7 représente l'autoradiographie d'un gel de polyacrylamide. Elle montre la détection du messager du gène de la β actine dans différentes cellules humaines ou de rat à l'aide d'une ribosonde (complémentaire à un 250 nucléotides du messager du gène de la β actine de souris) constituée de 15% de biotine-UTP et de 10% de DIG-UTP et radiomarquée au (α³²P)CTP. L'ordre des différentes cellules ou tissus testés est : cerveau (rat), rein (rat), foie (rat), Jurkat (humain), HepG2 (humain), H4II (rat), placenta (humain), Levure, Hela (humain).Pour chaque échantillon il a été réalisé un contrôle de la ribosonde sans Rnase T1 (-) et une détection du ARNm du gène de la β actine (+). Le contrôle de la spécificité de la réaction est réalisé à l'aide de la levure.

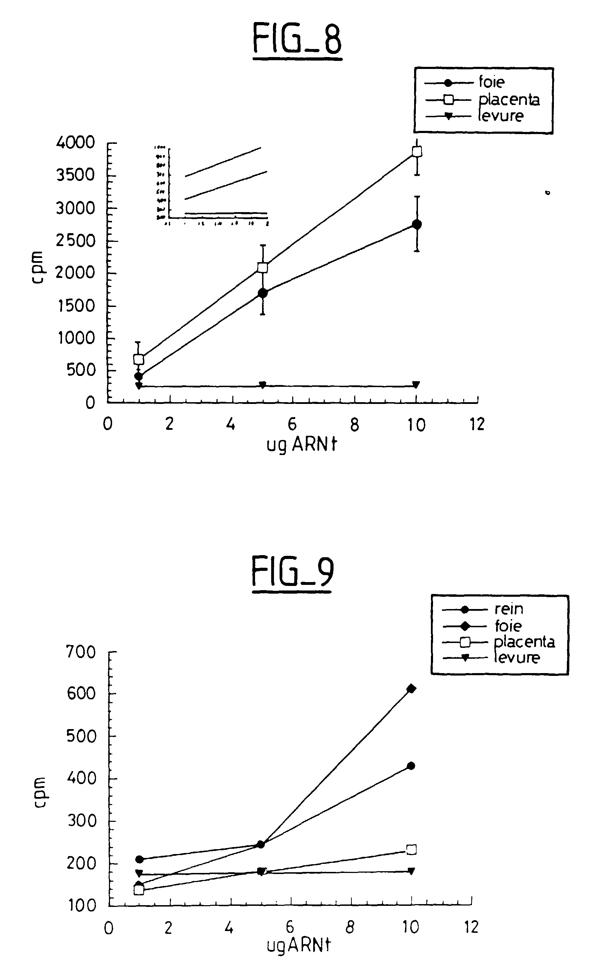

La figure 8 représente une détection sur plaque (après protection de la digestion à la ribonucléase T1) du messager de la β actine par une ribosonde constituée de 15% de biotine-UTP et de 10% de DIG-UTP, radiomarquée au (α³²P)CTP (3 10⁴ cpm/fmole).

La détection de la présence de la ribosonde est réalisée après découpage des puits et comptage de la radioactivité sur un compteur LS6000IC Beckman. L'ARNm de la β actine est recherché dans 10 µg, 5 µg et 1 µg des ARN totaux extrait à partir de différents tissus (voir légende).

La figure 9 représente une détection sur plaque d'un messager rare, celui de HNF1 (Facteur Nucléaire Hépatique 1) à l'aide d'une ribosonde (15% biotine-UTP,10% DIG-UTP) radiomarquée au α³²P-CTP (5 10⁴ cpm/fmole). Les échantillons testés correspondent à 10, 5 et 1 µg de ARNt provenant de rein, foie, placenta et levure.

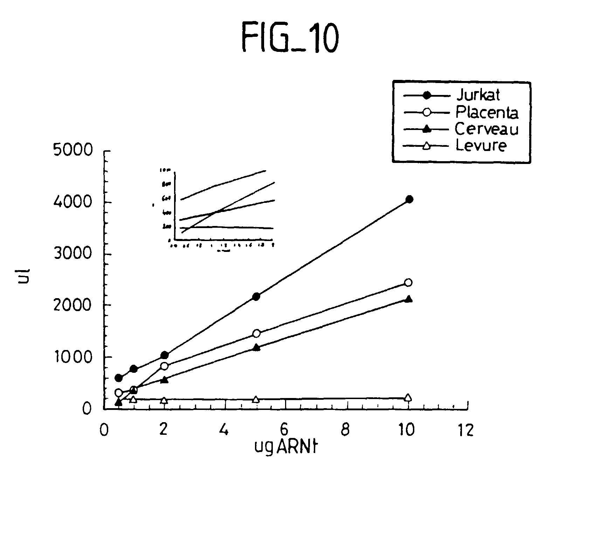

La figure 10 représente une détection sur plaque du messager de la β actine par une ribosonde constituée de 15% de biotine-UTP et de 10% de DIG-UTP. La détection de la ribosonde est réalisée à l'aide d'un anticorps anti-DIG couplé à la phosphatase alcaline. La mesure est réalisée en luminescence.

Dans les exemples on utilise des ribosondes modifiées obtenues par transcription à partir de monomères nucléotidiques nommés NTP comprenant des nucléotides modifiées UTP-biotine (Boehringer, Réf. 1388908) et UTP-DIG (Boehringer Réf. 1209256).

Lorsque l'on dit que la ribosonde est obtenue à partir de n% de biotine-UTP ou DIG-UTP, cela signifie que le pourcentage de biotine-UTP par rapport à la quantité totale d'UTP modifié et non modifié utilisé pour la transcription est de n%. Ceci correspond à un pourcentage de nucléotide modifié correspondant dans la ribosonde obtenue d'environ n/4%.

L'exemple 1 montre l'aspect bifonctionnelle des ribosondes biotine-UTP et DIG-UTP. L'exemple 2 souligne l'importance du taux de modification au niveau du test de protection de la digestion par les ribonucléases RPA. L'exemple 3 illustre une application de l'invention comme méthode de détection de l'expression des gènes.

### EXEMPLE 1 : MISE EN ÉVIDENCE DE LA CAPTURE ET DE LA DÉTECTION D'UNE RIBOSONDE CONSTITUÉE DE BIOTINE-UTP ET DE DIG-UTP SUR UNE MICROPLAQUE.

Avec les projets concernant le séquençage du génome humain un effort considérable a été effectué au niveau de l'automatisation des méthodes de séquençage de l'ADN. Cependant, les méthodes classiques d'analyses de l'expression des gènes ne sont pas adaptées à la détection d'un nombre de plus en plus croissant de messagers. Ainsi un avantage majeur d'une nouvelle méthode de détection des ARN messagers réside dans sa possibilité d'automatisation. Et doit pouvoir être réalisée sur un support solide comme une microplaque qui est particulièrement adaptée à l'étude de grandes séries d'échantillons. L'utilisation d'une ribosonde possédant une partie fonctionnelle permettant d'une part sa capture et possédant une propriété de reconnaissance spécifique apparaît bien adaptée à la détection des ARN messagers. Dans l'exemple présenté la capture est faite par la biotine et la détection par la DIG.

### 1. Principe général de l'utilisation d'une ribosonde.

Un segment d'ADN correspondant à une partie d'un gène est inséré au niveau d'un site de clonage immédiatement en aval d'un promoteur de l'ARN polymérase de bactériophage (T3, T7 ou SP6), dans une orientation qui conduit à la production d'un ARN antisens. Le plasmide recombinant est ensuite coupé par une enzyme de restriction en 3' de l'insert. Le plasmide linéarisé est alors transcrit en présence de ribonucléotides comprenant des ribonucléotides modifiés par exemple la biotine-UTP et la DIG-UTP. Un excès de cet ARN doublement marqué est hybridé en solution avec les ARN m à tester. Les hybridations sont réalisées dans des conditions stringentes standards à T≈40-50°C, durant la nuit (16 heures) dans un tampon 80 % formamide NaCl 0,4M à pH entre 7 et 8. La sonde non hybridée est ensuite supprimée par digestion à l'aide des ribonucléases spécifiques de l'ARN simple brin comme les RNases CL3, T1,Phy M,U2 ou A. La présence de la première modification, par exemple la biotine-UTP, dans l'hybride ARNm:ribosonde (biotine-DIG) permet sa capture sur une microplaque de titration dont la surface est recouverte de streptavidine. La présence d'une autre modification, par exemple la DIG, permet la détection et la quantification de l'hybride par une méthode ELISA utilisant un anticorps anti-DIG couplé à la phosphatase alcaline. Par ailleurs il est également possible de réaliser une transcription en présence de (α³²P)CTP afin de pouvoir détecter et quantifier l'hybride (ARNm:ribosonde) après dépôt sur plaque.

### 2. Système modèle : HNF1 (Hepatic Nuclear Factor 1).

Un fragment Pvull de 490 pb d'un ADNc codant pour HNF1 de rat (992 à 1482, Chouard et al 1990, Nucleic Acids Research, 18, 5853-5863) est sous cloné au site SmaI du vecteur pBS (Stratagene). La ribosonde antisens est obtenue après linéarisation du vecteur par coupure avec EcoR1, suivi d'une transcription réalisée par la ARN polymérase T3. La synthèse de la ribosonde sens est accomplie après coupure du vecteur par HindIII, suivi d'une transcription par la ARN polymérase T7.

### 3. Synthèse de la ribosonde.

Chaque réaction de transcription avec la T7 ou la T3 ARN polymérase est réalisée dans un tube Eppendorf avec les conditions suivantes H2O DEPC q.s.p. 20 µl; 40 mM Tris-HCl pH 7,5; 6 mM MgCl2; 2 mM spermidine; 5 mM NaCl; 10 mM DTT; 100 µg/ml sérum albumine bovine (SAB) (fraction V, Sigma); 500 µM CTP, ATP, GTP; des quantités variables de UTP, biotine-UTP et DIG-UTP selon le pourcentage de modification souhaité (par exemple, pour obtenir une ribosonde 15% biotine et 10% DIG: 75 µM biotine-UTP; 50 µM DIG-UTP, 375 µM UTP); 1 U/ml d'inhibiteur de ribonucléase; 40 U de T7 ou T3 ARN polymérase; 1 µg ADN plasmidique linéarisé après digestion par une endonucléase de restriction appropriée (EcoR1 ou Hind III). Le mélange est incubé 2 heures à 37°C. La transcription terminée, l'ADN plasmidique est digéré en ajoutant 1 µl (1 unité) de DNase RQ1 (Promega). La réaction est incubée 15 minutes à 37°C. Les ribonucléotides non incorporés sont éliminés par passage sur une colonne de chromatographie d'exclusion Biospin30 (Biorad).

Afin de quantifier la synthèse de la ribosonde ou de déterminer radioactivement la présence d'un hybride (ARNm:ribosonde) la transcription est réalisée avec 1-7 µl de (a32P)CTP (800 Ci/ml; 20 mCi/ml), selon l'activité spécifique désirée. La qualité de la ribosonde est déterminée par migration sur un gel d'électrophorèse d'acrylamide 5% en conditions dénaturantes.

### 4. Capture des ribosondes sur microplaque.

Les puits de microplaques liés avec de la streptavidine (Boehringer 1602853) sont recouverts par 100 µl d'hybride dilué dans du tampon 1X PBS; 1% SAB (sérum albumine bovine) pendant 1 heure à 37°C. Les plaques sont ensuite lavées 5 fois avec du tampon de lavage : 0,1M NaCl; 0,1 M Tris HCl; 0,1% Tween 20; 3 mM MgCl2, pH 7,5. Puis 100 µl (70 mU) d'anticorps anti-DIG couplé à la phosphatase alcaline (Boehringer) sont additionnés et incubés 1 heure à 37°C dans du tampon de lavage contenant 1% SAB.

### 5. Détection des ribosondes sur microplaque.

Selon la modification de la sonde, la détection peut être froide (fluorescente ou luminescente) ou radioactive.

### 5.1. Détection fluorescente

La réaction est réalisée avec 100 µl de substrat 4-méthylumbelliféryl phosphate (Sigma) à 0,4 mg/ml dans du tampon diéthanolamine 0,1M, pH 9,8. L'incubation est faite pendant la nuit à température ambiante. La mesure de la fluorescence est faite par un fluorimètre (Dynatech) avec une longeur d'onde d'excitation de 365 nm et une longeur d'émission de 450 nm.

### 5.2. Détection luminescente

La réaction est réalisée avec 100 µl de substrat AMPPD (Tropix) dilué au 1/59 en présence d'amplificateur Sapphire (Tropix) au 1/10 dans du tampon diéthanolamine 0,1 M, pH 9,8. La mesure de la luminescence est faite après 20 minutes à température ambiante sur luminomètre Micro Beta Trilux (Wallac).

### 5.3 Détection radioactive

Elle implique l'utilisation de ribosondes radiomarquées. Après découpage, chaque cupule est mise en présence de 3 ml de liquide scintillant. La mesure de la radioactivité est réalisée sur un compteur LS6000IC Beckman.

### 6. Résultats

De la biotine-UTP a été incorporée au niveau de la ribosonde, ce qui lui permet d'être capturée de façon spécifique par de la streptavidine préalablement immobilisée sur une microplaque

Pour toutes les études de fixation sur plaque on a utilisé comme contrôle de spécificité une ribosonde radiomarquée ne possèdant pas de biotine-UTP dans sa séquence.

### 6.1 Influence du pourcentage de biotine sur le taux de capture d'une ribosonde simple brin.

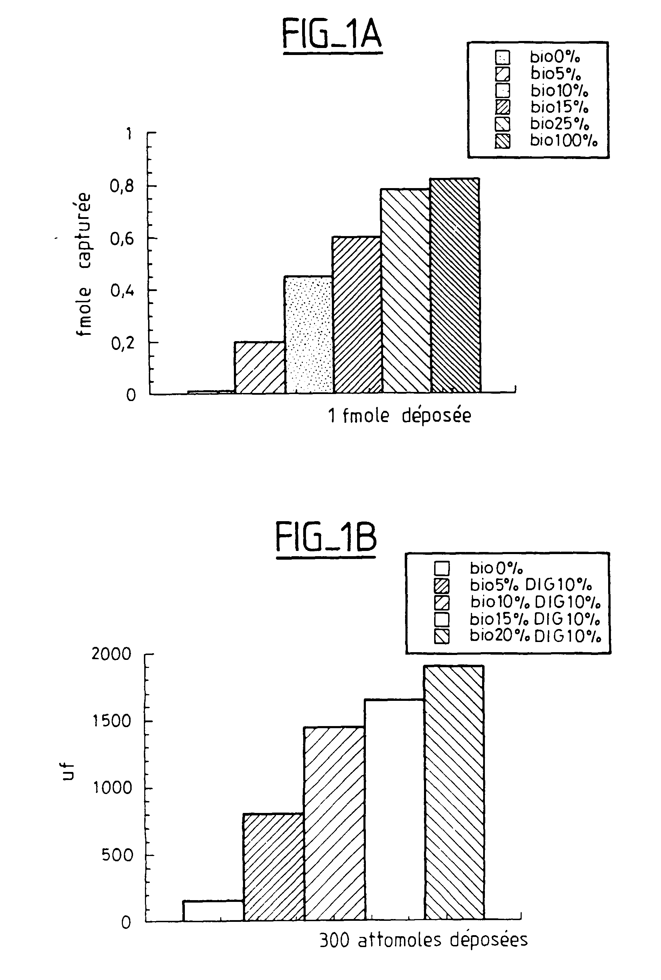

Comme le montre la Figure 1A, la capture de la ribosonde simple brin (biotine-UTP, (a32P) CTP) est corrélée à la quantité de biotine incorporée dans la ribosonde. La plus faible fixation a été obtenue avec 5% de biotine. Puis augmente de façon linéaire jusqu'à 25% de biotine, pour arriver à un plateau. La détection est faite par mesure de la radioactivité. Lorsqu'on utilise une ribosonde comportant 100% de biotine-UTP, le taux de capture maximale est de 80%.

L'influence du pourcentage de biotine-UTP sur le taux de capture peut également être décelée par un système de détection froid. Cependant, il est nécessaire d'incorporer de la DIG-UTP dans la ribosonde. La Figure 1B montre un tel système de détection. En accord avec les résultats précédants, le taux de capture augmente avec le pourcentage de biotine-UTP.

### 6.2 Seuil de sensibilité des différents systèmes de détection.

Différents types de détection peuvent être utilisés, radioactive ou froide.

La détection radioactive est réalisée après l'incorporation d'un ribonucléotide radiomarqué par le (a32P) UTP ou le (a32P) CTP.

La détection froide est possible grâce à l'incorporation d'un nucléotide modifié tel que la biotine, la digoxigénine, ou la fluorescéine, détecté par un conjugué constitué d'un ligand spécifique (la streptavidine pour la biotine) ou d'un anticorps spécifique (anti-biotine, anti-DIG, anti-fluorescéine) couplé avec une enzyme (phosphatase alcaline, peroxidase). La révélation est faite par la mesure de l'apparition d'un produit fluorescent ou luminescent.

Dans la suite la détection est faite soit par l'incorporation d'un nucléotide radioactif (a32P) CTP, ou par l'incorporation de DIG-UTP suivi d'une mesure de fluorescence ou-de chémoluminescence.

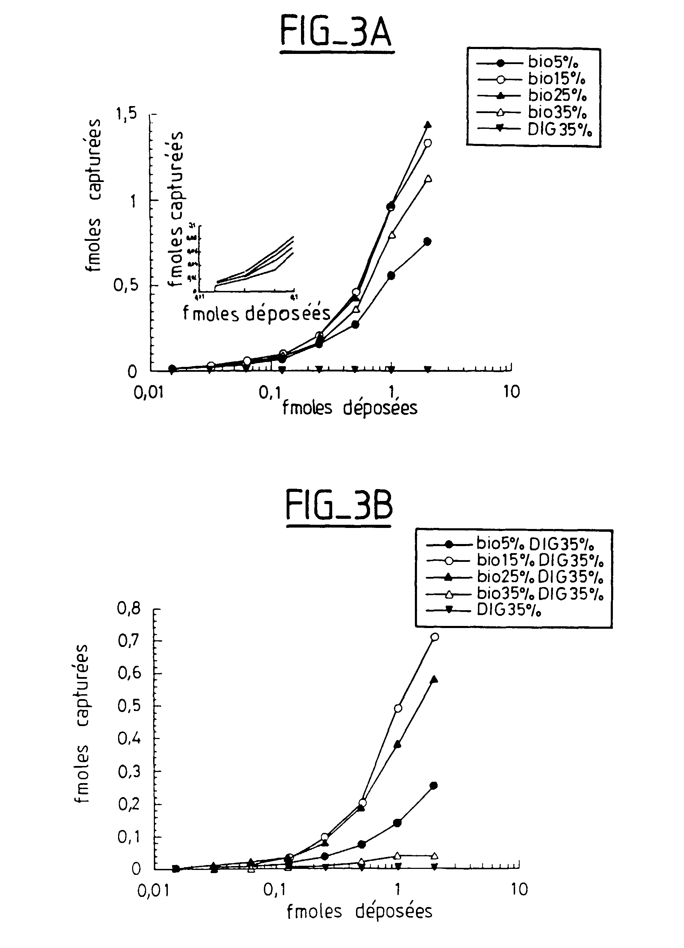

Dans le cas de la détection radioactive, pour obtenir la plus grande sensibilité possible, on a utilisé des ribosondes ayant une forte activité spécifique (2 105 cpm/fmole). Dans ces conditions, la Figure 3A montre qu'il est possible de détecter jusqu'à 1 attomole de ribosonde sur la plaque.

Pour la détection froide, comme lors de la détection radioactive, le seuil de sensibilité dépend du niveau d'incorporation du nucléotide modifié servant à la détection (DIG-UTP).

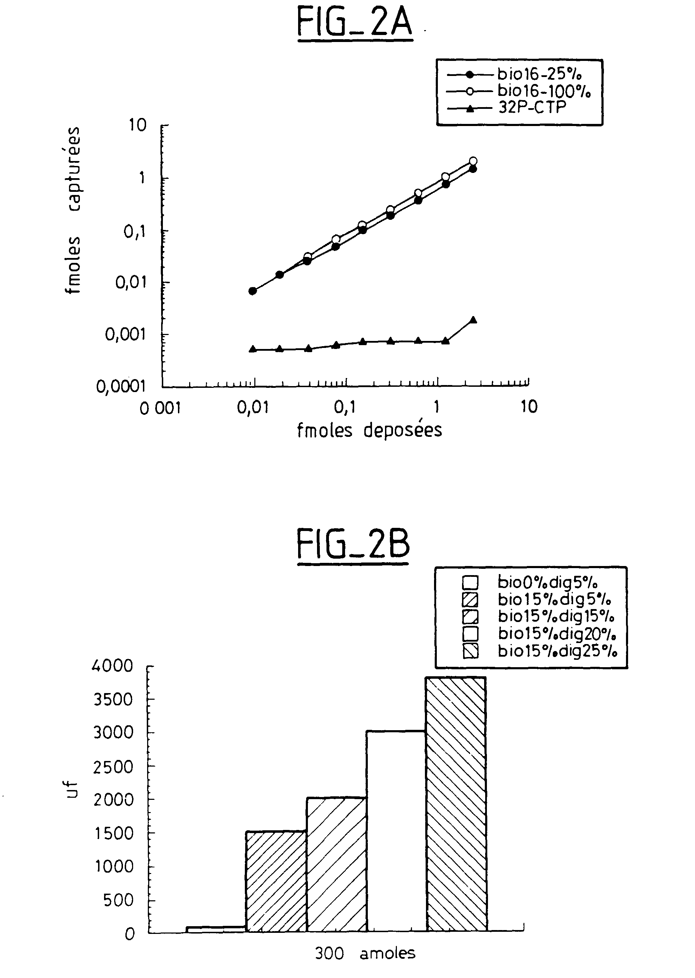

Par exemple, la figure 2B montre que l'intensité du signal augmente avec le pourcentage de DIG-UTP présent dans la ribosonde : une sonde constituée de 25 % de DIG-UTP est détectée 2,5 fois plus qu'une sonde ayant 5% de DIG-UTP. Dans des conditions d'incorporation de DIG-UTP correspondant au maximum de détection (35 % DIG-UTP) le seuil de détection est de 10 attomoles en fluorescence et de 5 attomoles en luminescence.

Ces résultats montrent qu'il est possible de détecter avec des systèmes différents (radioactif ou froid) des ribosondes capturées sur microplaque. Dans tous les cas le seuil de détection dépend du niveau de modification de la sonde.

### 6.3 Capture et détection des molécules d'ARN double brin.

Après avoir vérifié qu'il était possible de capturer et de détecter sur une microplaque une molécule d'ARN simple brin, on a étudié la possibilité de capture de molécules doubles brins. Effectivement, la présence d'un ARNm donné dans un échantillon est vérifiée par hybridation avec une ribosonde. L'hybride formé est protégé de la dégradation par les ribonucléases, alors que les molécules simples brins sont éliminées par digestion. C'est donc la détection de molécules hybrides qui révèle la présence d'un ARNm. Les molécules doubles brins étudiées comportent différent pourcentage de modifications.

La Figure 3A montre l'étude de la fixation des ribosondes (5,15,25,35% biotine-UTP) après formation d'un hybride avec une séquence ribonucléotidique complémentaire radiomarquée au (α³²P) CTP. Il est à noter qu'afin de pouvoir suivre le comportement sur plaque de l'hybride seul le brin complémentaire est radiomarqué.

Il apparaît que l'optimum de fixation de l'hybride se trouve entre 15 et 25% de biotine-UTP.

Comme pour la ribosonde simple brin 5% de biotine-UTP est insuffisant pour fixer correctement l'hybride.

La figure 3B montre l'influence du taux de biotine-UTP sur la capture d'un hybride constitué de (5, 15, 25, 35 %) biotine-UTP et de 35% DIG-UTP.

Le maximum de capture de l'hybride biotine-UTP, DIG-UTP est obtenu avec 15% de biotine-UTP.

Ces résultats confirment ceux obtenus précédemment. Par ailleurs, une sonde comportant 35% de biotine-UTP entraine une très faible fixation de l'hybride. Probablement en raison d'une plus faible stabilité du duplex obtenu en présence de 35% DIG-UTP. Ce résultat met en évidence qu'une trop forte modification de la ribosonde déstabilise l'hybride, et se traduit par une très faible capture.

Il est également possible de détecter les molécules d'ARN double brin à l'aide d'anticorps anti-DIG couplés à la phosphatase alcaline suivi d'une mesure de fluorescence (résultats non montrés).

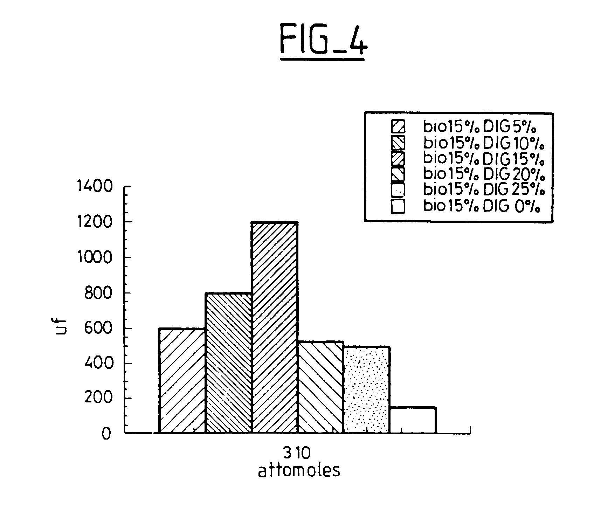

Par exemple, la figure 4 met en évidence que l'optimum de détection de l'hybride est atteint lorsque la ribosonde contenant 15 % de biotine-UTP comporte aussi 15 % de DIG-UTP.

L'ensemble des résultats indiquent une très bonne fixation de la ribosonde comportant 15% de biotine-UTP aussi bien sous forme simple brin que double brin.

Les résultats obtenus montrent la possibilité qu'ont les ribosondes biotine-UTP, DIG-UTP (sous forme simple et double brin) de se fixer et d'être détectées sur microplaque. Ces ribosondes possédant à la fois des propriétés de capture et de détection, apparaissent très adaptées à une détection automatisable des ARN messagers. Cependant on a observé un comportement différent de fixation sur plaque entre une molécule simple et double brin. Pour une molécule simple brin le taux de capture est lié directement à la quantité de biotine-UTP incorporée dans la ribosonde. Alors que pour une molécule double brin, le taux de capture passe par un optimum qui est atteint lorsque la ribosonde est constituée de 15% de biotine-UTP. Ainsi pour les molécules doubles brins, le taux de fixation n'est pas directement corrélé à la quantité de biotine contenue dans l'hybride, mais est une résultante entre la stabilité de l'hybride et le pourcentage de modification. Ceci implique la nécessité d'effectuer une optimisation de la fixation des molécules doubles brins.

### EXEMPLE 2 : EFFET DE L'INCORPORATION DANS UNE RIBOSONDE DE NUCLEODIDES MODIFIÉS SUR LA STABILITÉ À LA DÉGRADATION PAR DES RIBONUCLÉASES SPÉCIFIQUES DE SIMPLES BRINS.

L'exemple 1 montre qu'une trop forte proportion de modification entraine une déstabilisation des ARN doubles brins. Il est donc indispensable de connaître les conséquences de telles modifications au niveau de l'essai de protection de la digestion aux ribonucléases. Par ailleurs dans cet essai, l'appariement des deux brins de l'hybride doit être très stable, car tout désappariement entraîne une coupure par les ribonucléases et une perte du signal spécifique. D'autre part, les modifications se situant sur l'uridine, il est probable que les ribonucléases qui coupent au niveau de ce nucléotide soient moins tolérantes que celles qui coupent après un autre nucléotide.

Différents types de ribonucléases sont disponibles dans le commerce (C13, T1, A, PhyM, U2). Elles ont en commun de dégrader uniquement une molécule d'ARN sous forme simple brin et de laisser intactes les molécules double brins. Ces enzymes se distinguent par la spécificité de leurs sites de coupure.

Classiquement le test de protection aux ribonucléases est réalisé avec le mélange des ribonucléases A et T1. Cependant on a vérifié que la ribonucléase A qui coupe après les résidus uridine, cytidine et thymidine est moins utilisable que la ribonucléase T1 qui coupe seulement après la guanosine (non montré). Ceci a permis d'éliminer la ribonucléase A du test, et de garder uniquement la ribonucléase T1 qui offre un meilleur compromis entre la conservation du signal spécifique (molécule double brin) et la dégradation du signal non spécifique (simple brin). Afin d'éviter la présence d'un bruit de fond élevé lors de la révélation sur microplaque, il est nécessaire que la sonde non hybridée soit complètement digérée.

### 1. Synthèse des sondes

La synthèse des soudes et antisondes HNF1 a été réalisée comme dans l'exemple 1.

### 2. Hybridation

Une fmole de sonde antisens HNF1 comportant différentes modifications (biotine et/ou DIG) est mise à hybrider avec 10 et 1 ng de séquences complémentaires sens en présence de 10 µg d'ARN t de levure. Le tampon d'hybridation est constitué de 40 mM PIPES pH6,4; 1 mM EDTA pH8,0; 0,4 M NaCl; 80% formamide déionisé. Après dénaturation du mélange à 93°C pendant 4 minutes, l'hybridation est réalisée à 43°C pendant 16 heures.

### 3. Production de la digestion par la RNase T1

La digestion par la RNase T1 est réalisée après l'hybridation afin de dégrader la ribosonde et les ARNm non hybridés.

La digestion à la RNase T1 est réalisée en ajoutant 300 µl de tampon de digestion : 300 mM NaCl; 10 mM Tris HCl pH7,4; 5 mM EDTA pH7,5; 20 U RNase T1 (Boehringer). La digestion des ARN non hybridés est réalisée à 37°C pendant 30 minutes. Puis la RNase T1 est inactivée à l'aide de 20 µl d'une solution 10% SDS et 10 µl d'une solution de protéinase K à 10 mg/ml. Le mélange est incubé 30 minutes à 37°C. Les hybrides sont extraits avec 400 µl de mélange phénol:chloroforme:alcool isoamilique (Amesco). Après centrifugation à 12000 rpm pendant 5 minutes la phase supérieure est transférée dans un nouveau tube. Les hybrides sont précipités en présence de 200 µl d'acétate d'ammonium 4M et de 750 µl d'éthanol absolu pendant 30 minutes à -20°C.

Selon le protocole d'analyse des hybrides le culot est repris :
- soit dans 10 µl de tampon de charge comprenant 80% formamide; 0,1% xylène cyanol; 0,1% bleu de bromophénol; 2 mM EDTA. Puis le culot est analysé sur gel d'acrylamide en conditions dénaturantes,
- soit dans 100 µl de tampon de capture : 1X PBS; 1% albumine bovine sérique. Par la suite l'échantillon est analysé après fixation sur microplaque recouverte de streptavidine (Boehringer)

### 4. Analyse sur gel d'électrophorèse de polyacrylamide

Après dénaturation de l'échantillon par chauffage à 93°C pendant 4 minutes, celui-ci est soumis à une migration électrophorétique à 40-45V/cm dans un gel dénaturant à 5% de polyacrylamide; 7M urée contenant 1X TBE.

La radioactivité est détectée par autoradiographie. La taille de la bande correspondant à la ribosonde est déterminée par comparaison à la migration de fragments d'ADN de taille connue radiomarqués.

### 5. Résultats :

Les essais visaient à déterminer si les modifications de capture (biotine) ou de détection (DIG) de la ribosonde sont compatibles avec les tests de protection aux nucléases. Le test utilisé est une protection de la digestion par la RNase T₁, suivi par une analyse classique sur gel d'électrophorèse en conditions dénaturantes. La taille de la ribosonde HNF1 est de 500 nucléotides.

On a testé des ribosondes biotine-UTP (35 % et 100 %) (résultats non représentés) et DIG-UTP (5, 15, 20 et 35 %) (résultats représentés figure 5).

Il apparaît que, plus le pourcentage de modification augmente, moins la ribosonde est utilisable dans le test de protection aux ribonucléases. Ainsi, il n'est pas possible d'utiliser les ribosondes constituées de 35% de biotine-UTP et de 35% DIG-UTP, car il y a une perte complète du signal spécifique due à une dégradation par les ribonucléases, probablement en raison des contraintes qu'entrainent ces modifications au niveau de l'hybride. De la même façon, 100% de modification au niveau de UTP ne sont pas tolérés, et ceci quelle que soit la modification (biotine-UTP, DIG-UTP).

L'analyse fine de la dégradation de l'hybride en fonction du pourcentage de DIG-UTP incorporé dans la ribosonde révèle que l'augmentation du taux de modification entraîne une perte progressive de la sensibilité. Le seuil maximal de modification DIG-UTP sans perte significative de signal spécifique, est obtenu avec une sonde comportant 10 % de DIG-UTP (figure 5).

La figure 6 montre une étude comparative de la sensibilité entre une ribosonde (15% biotine-UTP, 10% DIG-UTP) et la même ribosonde non modifiée. Le test utilisé est une protection de la digestion par la Rnase T1, suivi par une analyse classique sur gel d'électrophorèse en conditions dénaturantes. Dans cette expérience les deux ribosondes radiomarquées possèdent une activité spécifique de 4.10⁴ cpm par fmole. La ribosonde (15% biotine-UTP, 10% DIG-UTP) est capable comme la sonde non modifiée de détecter 100 pg de séquence complémentaire mais avec un signal plus faible.

Ces résultats montrent que dans le cas de l'utilisation de la biotine comme modification de capture et DIG en détection, 15% de biotine et 10% de DIG sembent appropriés pour une bonne détection des ARN cibles. Avec d'autres modifications il, sera nécessaire de faire une optimisation.

### EXEMPLE - 3: UTILISATION D'UNE RIBOSONDE (15% BIOTINE-UTP, 10% DIG-UTP) POUR DÉTECTER LE MESSAGER DU GENE DE LA b ACTINE OU DE HNF1 SUR MICROPLAQUE.

La synthèse de la ribosonde HNF1, la capture et la détection ont été réalisées comme pour l'exemple 1.

### 1. synthèse de la sonde β actine

Un fragment de 250 pb d'un ADNc codant pour la β actine de souris (582-831) est cloné au site Kpnl/Xbal du vecteur pBS (Stratagene). La ribosonde β actine est obtenue après linéarisation du vecteur par coupure avec HindIII suivi d'une transcription par la ARN polymérase T7.

### 2. Extraction des ARN totaux

Les ARN totaux sont isolés à partir de 10⁷ cellules ou de 10 à 100 mg de tissus à l'aide de 2 ml de RNAzol B (Biotecx). Le tissu doit être plongé congelé dans le RNAzol B, l'homogénéisation se fait dans un broyeur "waring blender" pendant 30 à 60 secondes avant que le tissu ne décongèle, sinon les ARN seront dégradés par les Rnases. Puis 0,2 ml de chloroforme sont ajouté à l'homogénat. Après mélange et repos 5 minutes dans la glace, la solution est centrifugée à 12000 g pendant 15 minutes à 4°C. Après avoir prélevé la phase aqueuse un même volume d'isopropanol est ajouté. Le mélange est mis à précipiter à -20°C pendant 30 minutes. Après centrifugation à 12000 g pendant 15 minutes à 4°C le culot est lavé avec de l'alcool à 70%. Puis les ARN totaux sont resuspendus dans de l'eau DEPC. La concentration de l'échantillon est déterminée par mesure de la densité optique à 280 nm. La qualité de la préparation est vérifiée sur gel d'agarose 0,8%.

### 3. Hybridation

La quantité des ARN totaux nécessaire à la réaction d'hybridation dépend de la concentration de la séquence nucléotidique qui est recherchée et de l'activité spécifique de la ribosonde radiomarquée. Avec une ribosonde ayant une forte activité spécifique (>10⁹ cpm/µg) 10 µg de ARN totaux sont habituellement suffisant pour permettre la détection de ARNm qui sont présents à raison d'1 à 5 copies par cellule.

Les ARN totaux (10 µg) à analyser sont précipités en ajoutant 0,1 volume d'une solution 3 M d'acétate de sodium ph5,2 et 2,5 volumes d'éthanol froid. La solution est laissée 30 minutes à -20°C. Les ARNt sont récupérés par centrifugation à 12000 rpm pendant 15 minutes à 4°C. Puis le culot est lavé avec une solution d'éthanol à 70% et recentrifugé. Après élimination de l'éthanol, le culot est mis à sécher. Les ARNt sont ensuite dissous dans 20 ml de tampon d'hybridation contenant : 40 mM PIPES ph6,4; 1 mM EDTA ph8,0; 0,4 M NaCl; 80% formamide déionisé. Puis 1 fmole de la ribosonde biotine-DIG radiomarquée ou non est rajoutée. La solution est pipetée plusieurs fois pour permettre une solubilisation complète du culot. Le mélange est dénaturé par une incubation à 93°C pendant 4 minutes. Puis les tubes sont transférés rapidement dans un bain marie réglé à la température d'hybridation. Dans la plupart des cas, des résultats satisfaisants sont obtenus quand les ARN sont hybridés entre 45 et 50°C pendant 16 heures.

### 4. Résultats

La Figure 7 montre l'utilisation d'une ribosonde (15% biotine-UTP, 10% DIG-UTP,(α³²P)CTP) correspondant à une séquence complémentaire du messager de la β actine de la souris. La taille de la ribosonde est de 250 paires de bases.

La ribosonde (15% biotine-UTP, 10% DIG-UTP, (α³²P)CTP a été capable de détecter dans 10 µg de ARN totaux le messager de la β actine. Le test utilisé est une protection de la digestion par la Rnase T1 suivi d'une analyse par électrophorèse sur gel d'acrylamide en conditions dénaturantes. L'intégrité de la taille de la ribosonde est déterminée grâce à la présence de marqueurs radiomarqués.

La ribosonde apparaît protégée dans tous les échantillons testés (voir flèche sur la figure 7). Cependant il est à noter pour les échantillons humains la présence d'une deuxième bande de taille inférieure (flèche) qui est due à l'existence d'une différence dans la séquence du gène de la ββ-actine entre l'homme et la souris au niveau d'un site de coupure de la Rnase T1. Le messager de la β actine a été révélé dans toutes les cellules testées. La spécificité de la détection est donnée par la levure qui n'exprime pas de β actine.

Ceci démontre que la ribosonde (15% biotine-UTP, 10% DIG-UTP) s'apparie correctement avec une séquence complémentaire située dans un ARN m. Et qu'elle est donc utilisable dans les tests fonctionnels de protection de la digestion par la RNase T1.

La figure 8 montre les résultats obtenus après protection de la digestion par la Rnase T1 suivie d'une détection de la radioactivité après capture sur microplaque. La même quantité de ribosonde β actine (15% biotine-UTP,10% DIG-UTP) 1 fmole (4 10⁴ cpm) a été utilisée pour la détection sur gel et sur plaque. Comme dans l'analyse par gel d'électrophorèse de polyacrylamide la ribosonde (15% biotine-UTP, 10% DIG-UTP, (α³²P)CTP) est capable de détecter la présence du messager de la β actine à partir d'1 µg de ARN totaux. La levure utilisée comme témoin négatif de réaction donne un bruit de fond très bas (250 cpm) comparé aux 1800-4000 cpm obtenus avec 5 et 10 µg de ARNt de cellules exprimant la β actine. Bien que beaucoup plus faible (400-680 cpm) le signal reste positif avec 1 µg de ARNt.

La figure 9 montre les résutats obtenus avec une ribosonde (500 nucléotides) dirigée contre un messager faiblement exprimé, celui de HNF1. La sonde utilisée comportant 15% de biotine-UTP et 10% de DIG-UTP est radiomarquée avec du (α³²P)CTP (5 105⁵cpm/fmole). La mesure de la radioactivité associée avec les deux cellules exprimant HNF1 (rein et foie) est significativement plus forte que celle associée aux échantillons qui n'expriment pas HNF1 (placenta et levure). Ce résultat démontre la possibilité d'une détection par une ribosonde (biotine-UTP, DIG-UTP) d'un ARN m faiblement représenté.

Cependant pour être tout à fait automatisable cette méthode d'analyse sur microplaque doit s'affranchir de la radioactivité.

La figure 10 montre les résultats obtenus avec une ribosonde β actine (15% biotine-UTP, 10% DIG-UTP) suivi d'une détection sur microplaque par un anticorps anti-DIG, et une lecture en luminescence.

Les cellules testées sont : Jurkat, Placenta, Cerveau, Levure.

A partir de cellules Jurkat il a été possible de détecter le messager de la β actine dans 0,5 µg d'ARNt. Pour le placenta et le cerveau le messager de la β actine a été mis en évidence dans 2 µg d'ARNt. Ces résultats indiquent la très bonne sensibilité de la méthode de détection.

Ainsi comme en radioactivité la ribosonde est capable de détecter le messager de la β actine à partir de 10, 5, 2 ou 0,5 µg d'ARN totaux.

Des résultats similaires sont obtenus avec une détection fluorescente (non montré).

L'ensemble de ces travaux montrent que les ribosondes (biotine-UTP, DIG-UTP) tout en gardant leur spécificité de reconnaissance par l'intermédiaire de leurs séquences ribonucléotidiques sont capables de se fixer sur plaque et d'être détectées specifiquement. Leur utilisation dans des tests de protection aux nucléases a nécessité un ajustement du taux de leurs modifications.

Bien que la technique de protection aux nucléases soit fréquement utilisée dans les études de détection et de quantification des ARNm. Elle reste néanmoins peu adaptée à l'analyse de grandes séries d'échantillons. Car les produits des expériences de protection aux nucléases sont habituellement analysés sur gel de polyacrylamide en conditions dénaturantes. La possibilité de détecter sur microplaque des ARN messagers à l'aide de ribosonde (biotine-UTP, DIG-UTP) constitue un outil d'une très grande puissance d'analyse, et rend la technique de protection aux nucléases applicable à l'étude des grand nombres.

## Revendications

1. Procédé de détection d'acides nucléiques caractérisé en ce qu'il comporte les étapes suivantes :
a) on réalise l'hybridation en solution d'une sonde d'acide nucléique capable de s'hybrider avec un ARN ou un ADN donné dans un échantillon contenant des acides nucléiques, ladite sonde comprenant des éléments de détection et des premiers éléments de capture sur support solide ;
b) on réalise la dégradation des séquences nucléotidiques non hybridées dans l'échantillon par un enzyme dégradant les acides nucléiques non hybridées,
c) on effectue la capture des hybrides obtenus à l'étape b) en les mettant en présence d'un support solide revêtu de deuxièmes éléments de capture interagissant avec lesdits premiers éléments de capture de ladite sonde, et
d) on effectue la détection des hybrides ou des sondes provenant des hybrides après dénaturation par l'intermédiaire desdits éléments de détection.

2. Méthode de détection d'ARN messagers selon la revendication 1, caractérisée en ce qu'à l'étape a) on utilise une sonde d'ARN et à l'étape b) on réalise la dégradation enzymatique avec une RNase.

3. Méthode selon la revendication 1 ou 2, caractérisée en ce que lesdits premiers éléments de capture de ladite sonde sont constitués par une première molécule biologique fixée de façon covalente à ladite sonde et lesdits deuxièmes éléments de capture dudit support solide sont constitués par une deuxième molécule biologique fixée sur le support solide, deuxième molécule biologique qui interagit et se lie de façon non covalente avec ladite première molécule.

4. Méthode selon l'une des revendications 1 à 3, caractérisée en ce que pour détecter un ARNm exprimé à partir d'un gène donné, on utilise une sonde d'ARN obtenue par transcription in vitro de fragments d'ADNc clonés dudit gène.

5. Méthode selon l'une des revendications 1 à 4, caractérisée en ce qu'on utilise une sonde d'ARN obtenue par transcription in vitro à l'aide de nucléotides dont certains sont modifiés par couplage à l'un desdits éléments de détection et/ou l'un desdits premiers éléments de capture.

6. Méthode selon l'une des revendications 1 à 5, caractérisée en ce que la sonde comporte de 100 à 1000 nucléotides, de préférence de 250 à 500 nucléotides.

7. Méthode selon l'une des revendications 1 à 6, caractérisée en ce qu'après l'étape b) on élimine les produits de dégradation des acides nucléiques non hybridés.

8. Méthode selon l'une des revendications 1 à 7, caractérisé en ce que la sonde ne comporte pas plus de 15 %, de préférence pas plus de 10 % de nucléotides modifiés par lesdits éléments de détection et lesdits premiers éléments de capture constitués par des molécules biologiques.

9. Méthode selon l'une des revendications 1 à 8, caractérisée en ce que lorsque les éléments de détection et lesdits premiers éléments de capture sont substitués sur des nucléotides donnés, on utilise à l'étape b) un enzyme qui coupe les acides nucléiques simple brin au niveau d'un nucléotide autre que lesdits nucléotides substitués.

10. Méthode selon la revendication 9, caractérisée en ce que les éléments de détection et lesdits premiers éléments de capture sont substitués sur des nucléotides d'Uridine et l'enzyme de l'étape b) est une RNase T₁.

11. Méthode selon l'une des revendications 3 à 10, caractérisée en ce que lesdites première et deuxième molécules de capture constituent un couple biotine / streptavidine.

12. Méthode selon l'une des revendications 3 à 10, caractérisée en ce que ladite première molécule de capture est la digoxigénine (DIG) et ladite deuxième molécule de capture est un anticorps anti-digoxigénine.

13. Méthode selon les revendications 1 à 12, caractérisée en ce que ledit élément de détection est un élément directement détectable, radioactif ou fluorescent.

14. Méthode selon la revendication 1 à 12 caractérisée en ce que ledit élément de détection est un élément indirectement détectable et qu'il est constitué par une molécule biologique susceptible de réagir de manière non covalente avec une autre molécule comportant un élément directement détectable.

15. Méthode selon la revendication 13 caractérisée en ce que l'élément de détection indirectement détectable est choisi parmi la biotine, la fluoresceine et la digoxigénine.

16. Méthode selon l'une des revendications 1 à 15, caractérisée en ce que ledit premier élément de capture est choisi parmi la biotine, la fluoresceine et la digoxigénine.

17. Méthode selon l'une des revendications 1 à 16 caractérisée en ce que l'élément de détection est la digoxigénine et/ou la biotine et ledit premier élément de capture est choisi respectivement parmi la biotine et la digoxigénine.

18. Procédé selon la revendication 17, caractérisé en que la sonde comporte de 2 à 5%, notamment 3 à 4%, de nucléotides substitués par une molécule de biotine et de 2 à 5% notamment 2 à 3% de nucléotides substitués par une molécule de digoxigénine.

19. Méthode de diagnostic dans laquelle on met en oeuvre une méthode de détection selon l'une des revendications 1 à 18 dans laquelle on détecte un acide nucléique impliqué dans une pathologie.

20. Kit utile pour la mise en oeuvre d'un procédé selon l'une des revendications 1 à 19, caractérisé en ce qu'il comporte des sondes d'acide nucléique de taille importante, notamment de plus de 100 nucléotides, portant les éléments de détection et lesdits premiers éléments de capture sur support solide et un support solide revêtu desdits deuxièmes éléments de capture.

## Claims

1. Method for detecting nucleic acids characterized in that it entails the following steps:
a) hybridization is carried out in solution of a nucleic acid probe capable of hybridizing with a given RNA or DNA in a sample containing nucleic acids, the said probe comprising detection components and first capture components on a solid support;
b) degradation of the unhybridized nucleotide sequences in the sample is carried out with an enzyme that degrades unhybridized nucleic acids,
c) capture of the hybrids obtained in step b) is accomplished by bringing them into contact with a solid support coated with second capture components that interact with the said first capture components of the said probe, and
d) detection of the hybrids, or of the probes originating from the hybrids after denaturation, is accomplished by means of the said detection components.

2. Method for detecting messenger RNAs according to Claim 1, characterized in that, in step a) an RNA probe is used, and in step b) the enzymatic degradation is carried out with an RNase.

3. Method according to Claim 1 or 2, characterized in that the said first capture components of the said probe consist of a first biological molecule bound covalently to the said probe and the said second capture components of the said solid support consist of a second biological molecule bound to the solid support, which second biological molecule interacts and binds non-covalently to the said first molecule.

4. Method according to one of Claims 1 to 3, characterized in that, to detect an mRNA expressed from a given gene, an RNA probe obtained by in vitro transcription of cloned cDNA fragments of the said gene is used.

5. Method according to one of Claims 1 to 4, characterized in that an RNA probe obtained by in vitro transcription with nucleotides, some of which are modified by coupling to one of the said detection components and/or one of the said first capture components, is used.

6. Method according to one of Claims 1 to 5, characterized in that the probe contains from 100 to 1000 nucleotides, preferably from 250 to 500 nucleotides.

7. Method according to one of Claims 1 to 6, characterized in that, after step b), the degradation products of the unhybridized nucleic acids are removed.

8. Method according to one of Claims 1 to 7, characterized in that the probe does not contain more than 15%, preferably not more than 10%, of nucleotides modified with the said detection components and the said first capture components consisting of biological molecules.

9. Method according to one of Claims 1 to 8, characterized in that, when the detection components and the said first capture components are substituted on given nucleotides, an enzyme is used in step b) which cuts the single-stranded nucleic acids at a nucleotide other than the said substituted nucleotides.

10. Method according to Claim 9, characterized in that the detection components and the said first capture components are substituted on Uridine nucleotides and the enzyme of step b) is an RNase T₁.

11. Method according to one of Claims 3 to 10, characterized in that the said first and second capture molecules constitute a biotin/streptavidin system.

12. Method according to one of Claims 3 to 10, characterized in that the said first capture molecule is digoxigenin (DIG) and the said second capture molecule is an anti-digoxigenin antibody.

13. Method according to Claims 1 to 12, characterized in that the said detection component is a directly detectable radioactive or fluorescent component.

14. Method according to Claim 1 to 12, characterized in that the said detection component is an indirectly detectable component and in that it consists of a biological molecule capable of reacting noncovalently with another molecule containing a directly detectable component.

15. Method according to Claim 13, characterized in that the indirectly detectable detection component is chosen from biotin, fluorescein and digoxigenin.

16. Method according to one of Claims 1 to 15, characterized in that the said first capture component is chosen from biotin, fluorescein and digoxigenin.

17. Method according to one of Claims 1 to 16, characterized in that the detection component is digoxigenin and/or biotin and the said first capture component is chosen from biotin and digoxigenin, respectively.

18. Method according to Claim 17, characterized in that the probe contains from 2 to 5%, in particular 3 to 4%, of nucleotides substituted with a biotin molecule and from 2 to 5%, in particular 2 to 3%, of nucleotides substituted with a digoxigenin molecule.

19. Diagnostic method in which a detection method according to one of Claims 1 to 18 is employed, in which a nucleic acid involved in a pathology is detected.

20. Kit which is useful for carrying out a method according to one of Claims 1 to 19, characterized in that it contains large nucleic acid probes, in particular ones having more than 100 nucleotides, carrying the detection components and the said first capture components on a solid support, and a solid support coated with the said second capture components.

## Patentansprüche

1. Verfahren zum Nachweis von Nukleinsäuren, dadurch charakterisiert, daß es die folgenden Schritte umfaßt:
a) man führt die Hybridisierung einer Nukleinsäuresonde in Lösung durch, die sich mit einer gegebenen RNA oder DNA in einer Nukleinsäuren enthaltenden Probe hybridisieren kann, wobei die Sonde Nachweiselemente und erste Abfangelemente auf einem festen Träger umfaßt;
b) man führt den Abbau der nicht hybridisierten nukleotidischen Sequenz in der Probe mit einem Enzym durch, das die nicht hybridisierten Nukleinsäuren abbaut,
c) man führt das Abfangen der in Schritt b) erhaltenen Hybride durch, indem man sie in Kontakt mit einem festen Träger bringt, der mit zweiten Abfangelementen versehen ist, die mit den ersten Abfangelementen der Sonde wechselwirken, und
d) man führt den Nachweis der Hybride oder der aus den Hybriden stammenden Sonden nach Denaturierung durch Vermittlung der Nachweiselemente durch.

2. Verfahren zum Nachweis von messenger-RNA nach Anspruch 1, dadurch charakterisiert, daß man in Schritt a) eine RNA-Sonde verwendet und in Schritt b) den enzymatischen Abbau mit einer RNase durchführt.

3. Verfahren nach Anspruch 1 oder 2, dadurch charakterisiert, daß die ersten Abfangelemente der Sonde gebildet werden aus einem ersten biologischen Molekül, das kovalent an die Sonde gebunden ist, und daß die zweiten Abfangelemente des festen Trägers gebildet werden aus einem zweiten biologischen Molekül, das an den festen Träger gebunden ist; ein zweites biologisches Molekül, das in nicht kovalenter Weise an das erste Molekül bindet und damit in Wechselwirkung tritt.

4. Verfahren nach einem der Ansprüche 1 bis 3, dadurch charakterisiert, daß man zum Nachweis einer ausgehend von einem gegebenen Gen exprimierten RNA eine RNA-Sonde verwendet, die durch in vitro-Transkription von klonierten cDNA-Fragmenten dieses Gens erhalten wurde.

5. Verfahren nach einem der Ansprüche 1 bis 4, dadurch charakterisiert, daß man eine RNA-Sonde verwendet, die durch in vitro-Transkription mit Hilfe von Nukleotiden erhalten wurde, von denen bestimmte durch Kopplung an eines der Nachweiselemente und/oder der ersten Abfangelemente modifiziert wurden.

6. Verfahren nach einem der Ansprüche 1 bis 5, dadurch charakterisiert, daß die Sonde 100 bis 1000 Nukleotide, bevorzugt 250 bis 500 Nukleotide umfaßt.

7. Verfahren nach einem der Ansprüche 1 bis 6, dadurch charakterisiert, daß man nach Schritt b) die Abbauprodukte der nicht hybridisierten Nukleinsäuren entfernt.

8. Verfahren nach einem der Ansprüche 1 bis 7, dadurch charakterisiert, daß die Sonde nicht mehr als 15 %, bevorzugt nicht mehr als 10 % Nukleotide umfaßt, die durch die Nachweiselemente und die ersten Abfangelemente, die aus biologischen Molekülen gebildet sind, modifiziert sind.

9. Verfahren nach einem der Ansprüche 1 bis 8, dadurch charakterisiert, daß man, wenn die Nachweiselemente und die ersten Abfangelemente an gegebenen Nukleotiden substituiert sind, man im Schritt b) ein Enzym verwendet, das die einzelsträngigen Nukleinsäuren bei einem Nukleotid schneidet, das von den substituierten Nukleotiden verschieden ist.

10. Verfahren nach Anspruch 9, dadurch charakterisiert, daß die Nachweiselemente und die ersten Abfangelemente an Uridin-Nukleotiden substituiert sind und das Enzym in Schritt b) eine RNase T₁ ist.

11. Verfahren nach einem der Ansprüche 3 bis 10, dadurch charakterisiert, daß das erste und zweite Abfangmolekül ein Paar Biotin/Streptavidin darstellt.

12. Verfahren nach einem der Ansprüche 3 bis 10, dadurch charakterisiert, daß das erste Abfangmolekül Digoxigenin (DIG) ist und daß das zweite Abfangmolekül ein anti-Digoxigenin-Antikörper ist.

13. Verfahren nach einem der Ansprüche 1 bis 12, dadurch charakterisiert, daß das Nachweiselement ein direkt nachweisbares, radioaktives oder fluoreszierendes Element ist.

14. Verfahren nach den Ansprüchen 1 bis 12, dadurch charakterisiert, daß das Nachweiselement ein indirekt nachweisbares Element ist und daß es gebildet wird aus einem biologischen Molekül, das in nicht kovalenter Weise mit einem weiteren Molekül reagieren kann, das ein direkt nachweisbares Element umfaßt.

15. Verfahren nach Anspruch 13, dadurch charakterisiert, daß das indirekt nachweisbare Nachweiselement ausgewählt wird aus Biotin, Fluorescein und Digoxigenin.

16. Verfahren nach einem der Ansprüche 1 bis 15, dadurch charakterisiert, daß das erste Abfangelement ausgewählt wird aus Biotin, Fluorescein und Digoxigenin.

17. Verfahren nach einem der Ansprüche 1 bis 16, dadurch charakterisiert, daß das Nachweiselement Digoxigenin und/oder Biotin ist und das das erste Abfangelement jeweils aus Biotin bzw. Digoxigenin ausgewählt wird.

18. Verfahren nach Anspruch 17, dadurch charakterisiert, daß die Sonde 2 bis 5 %, insbesondere 3 bis 4 % mit einem Biotinmolekül substituierte Nukleotide und 2 bis 5 %, insbesondere 2 bis 3 % mit einem Digoxigenin-Molekül substituierte Nukleotide umfaßt.

19. Diagnostisches Verfahren, bei dem man eine Nachweismethode nach einem der Ansprüche 1 bis 18 durchführt, bei der man eine in einem pathologischen Zustand involvierte Nukleinsäure nachweist.

20. Kit, das für die Durchführung eines Verfahrens nach einem der Ansprüche 1 bis 19 verwendbar ist, dadurch charakterisiert, daß es Nukleinsäuresonden von bedeutender Größe umfaßt, insbesondere mit mehr als 100 Nukleotiden, die die Nachweiselemente und die ersten Trägerelemente auf einem festen Träger tragen, und einen festen Träger, der mit den zweiten Nachweiselementen versehen ist.
